# EUROPEAN PATENT SPECIFICATION

(11) **EP 2 869 796 B1**
(45) Date of publication and mention of the grant of the patent: **09.08.2017**
(21) Application number: 13813486.1
(22) Date of filing: 27.06.2013
(51) Int. Cl.: A61F 5/02, A61F 7/02, A61F 5/055

(54) **PATIENT TREATMENT SYSTEM**
PATIENTENBEHANDLUNGSSYSTEM
SYSTÈME DE TRAITEMENT DE PATIENT

(30) Priority: 03.07.2012 US 201261667529 P
(43) Date of publication of application: 13.05.2015
(73) Proprietor: Medical Science Technologies LLC, Lawrence, NY 11559 (US)
(72) Inventor: HEYMAN, Frederic, Lawrence, NY 11559 (US)
(74) Representative: Schumacher & Willsau
(86) International application number: PCT/US2013/048203
(87) International publication number: WO 2014/008094

(56) References cited:
- US-A- 4 745 922
- US-A1- 2005 059 909
- US-A1- 2006 079 820
- US-A1- 2011 046 527
- US-A1- 2011 066 094
- US-A1- 2012 053 499
- US-A1- 2012 143 110

## Description

### BACKGROUND OF THE INVENTION

### 1. Field of the Invention

The invention relates to a patient treatment system. Specifically, the invention relates to a system that includes a cervical collar and one more aids, which may include a cooling aid for cooling the blood flow to the brain, wherein the aids are held in place in the cervical collar by one or more retainers.

### 2. Description of the Related Art

A cervical collar, also called a neck brace, is an orthopedic device that supports the head and neck of a wearer and limits the movement of the head and neck in unintended directions. Cervical collar may find use in the sports field or in medicine. When worn by athletes, such as automobile race drivers, cervical collars prevent traumatic injury when rapid deceleration occurs. In medical patients, a cervical collar supports a patient's neck and head, limits the movement of the neck and head to prevent worsening an existing injury, or relieves chronic medical conditions typically related to compression of the spinal cord and/or nerves connected to the spinal cord caused by unintended movement of the patient's neck and/or head.

In certain situations, the wearer of the cervical collar may be advantageously served by also regulating the wearer's body temperature.

US 2012/0143110 A1 discloses a cervical collar having an opening located over a trachea of a patient. The collar can be provided with cooling packs.

### SUMMARY OF THE INVENTION

These and other criteria are met by the present invention as claimed. A patient treatment system for treating a patient includes a cervical collar having a first opening located over a trachea of the patient and a second opening located over a carotid artery of the patient. An aid is disposed in the second opening. The aid includes a base having a curved surface; the base is disposed proximal to a neck of the patient over the carotid artery. The aid also includes a first reacting agent and a second reacting agent. The first reacting agent and the second reacting agent create a thermic reaction when mixed.

The aid further includes a first container and a second container. The base is part of the first container including the base; the second container is disposed entirely within in the first container. The aid also includes an actuator pressed by a user and an initiator that in a first state seals the first reacting agent and the second reacting agent from each other. The initiator is responsive to the actuator moving to a second state permitting the first reacting agent and the second reacting agent to mix.

Other embodiments and combinations of embodiments are intended and all embodiments may be used in combination with any one other or multiple other embodiments.

### BRIEF DESCRIPTION OF THE DRAWINGS

Fig. 1 is a perspective view of a patient treatment system in accordance with one or more embodiments of the present invention.
Fig. 2 is a front view of the patient treatment system of Fig. 1.
Fig. 3 is a rear view of the patient treatment system of Fig. 1.
Fig. 4 is a right-side view of the patient treatment system of Fig. 1.
Fig. 5 is a left-side view of the patient treatment system of Fig. 1.
Fig. 6 is a top view of the patient treatment system of Fig. 1.
Fig. 7 is a bottom view of the patient treatment system of Fig. 1.
Fig. 8 is a perspective view of a substrate of the patient treatment system of Fig. 1 in accordance with one or more embodiments of the present invention.
Fig. 9 is a front view of the substrate of Fig. 8.
Fig. 10 is a rear view of the substrate of Fig. 8.
Fig. 11 is a left-side view of the substrate of Fig. 8.
Fig. 12 is a right-side view of the substrate of Fig. 8.
Fig. 13 is a top view of the substrate of Fig. 8.
Fig. 14 is a top view of the substrate of Fig. 8.
Fig. 15 is a perspective view of an aid for a patient treatment system of Fig. 1 in accordance with one or more embodiments of the present invention.
Fig. 16 is a front view of the aid of Fig. 15.
Fig. 17 is a bottom view of the aid of Fig. 15.
Fig. 18 is a top view of the aid of Fig. 15.
Fig. 19a is a schematic illustrating the positional advantage of the aid in accordance with one or more embodiments of the present invention.
Fig. 19b is a schematic contrasting the advantages of the prior art and the advantages of the present invention.
Fig. 20 is a detailed view of a retainer in accordance with one or more embodiments of the present invention.
Fig. 21 a is a cross-sectional view of an aid for a patient treatment system in accordance with one embodiment of the present invention.
Fig. 21b is an isometric view of an aid for a patient treatment system in accordance with one embodiment of the present invention.
Fig. 22a is a front view of a patient treatment system in accordance with one or more embodiments of the present invention.
Fig. 22b is a rear view of the patient treatment system of Fig. 22a.
Fig. 23a is a perspective view of an aid for a patient treatment system in accordance with one embodiment of the present invention.
Fig. 23b is an exploded view of the aid of Fig. 23a.
Fig. 23c is a cross-sectional view of the aid of Fig. 23a.

### DETAILED DESCRIPTION OF THE INVENTION

Reference will now be made in detail to several views of the invention that are illustrated in the accompanying drawings. Wherever possible, same or similar reference numerals are used in the drawings and the description to refer to the same or like parts or steps. The drawings are in simplified form and are not to precise scale. For purposes of convenience and clarity only, directional terms, such as top, bottom, left, right, up, down, over, above, below, beneath, rear, and front may be used with respect to the drawings. These and similar directional terms should not be construed to limit the scope of the invention in any manner. The words "connect," "couple," and similar terms with their inflectional morphemes do not necessarily denote direct and immediate connections, but also include connections through intermediate elements or devices.

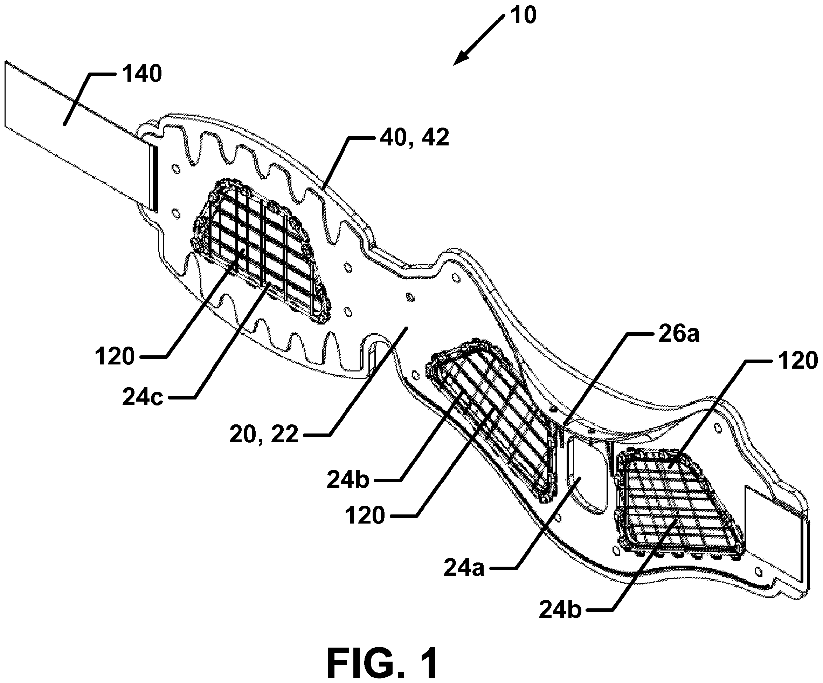

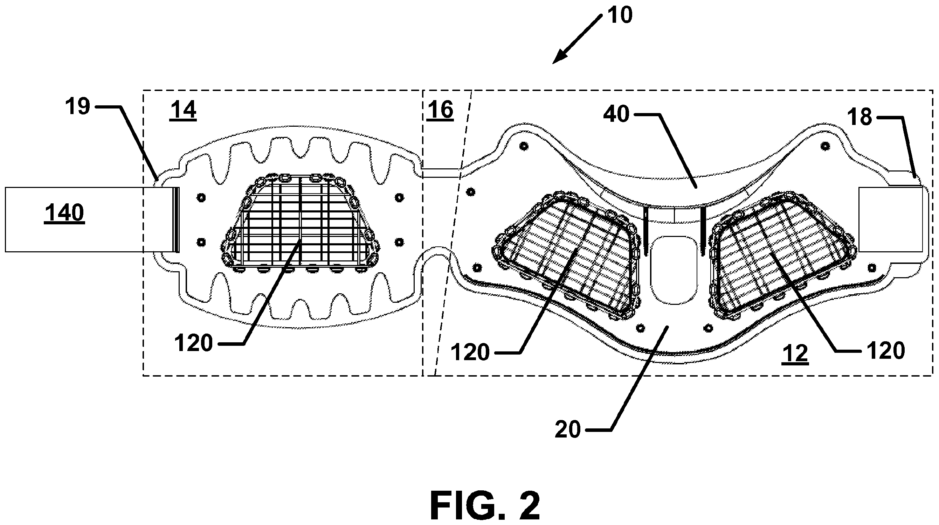

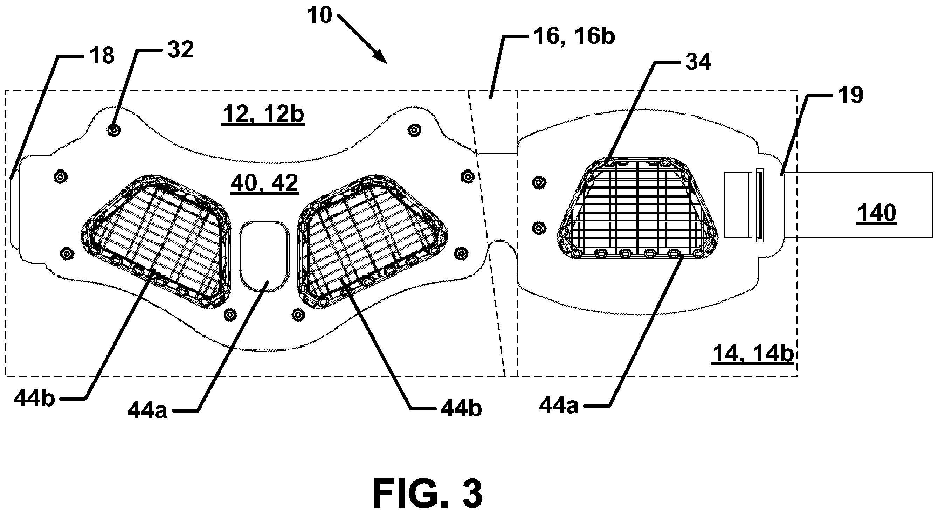

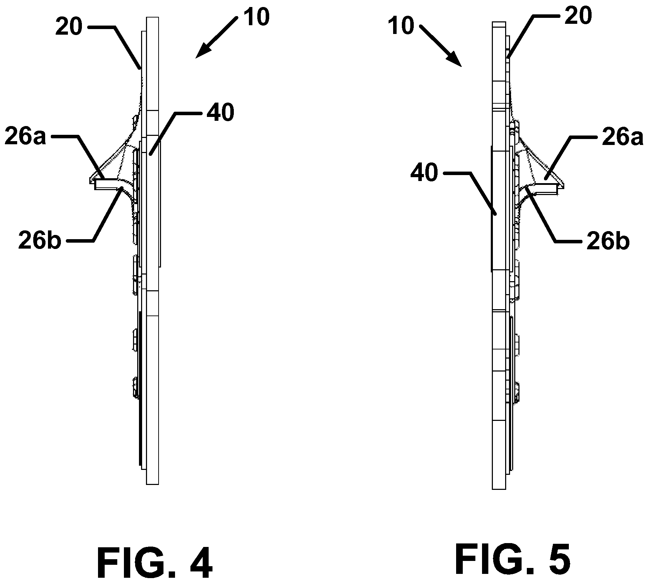

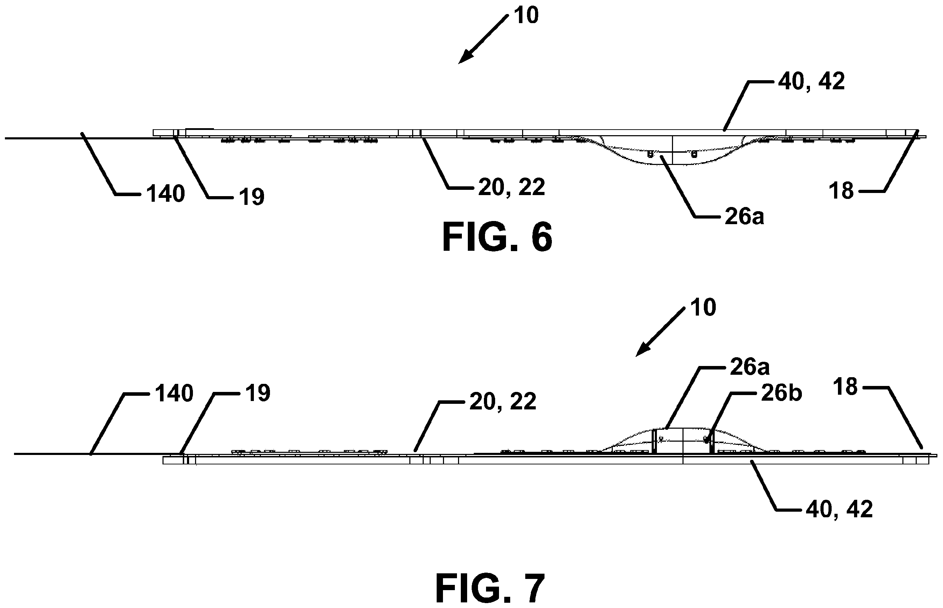

Fig. 1 is a perspective view of a patient treatment system in accordance with one or more embodiments of the present invention. Fig. 2 is a front view of the patient treatment system of Fig. 1. Fig. 3 is a rear view of the patient treatment system of Fig. 1. Fig. 4 is a right-side view of the patient treatment system of Fig. 1. Fig. 5 is a left-side view of the patient treatment system of Fig. 1. Fig. 6 is a top view of the patient treatment system of Fig. 1. Fig. 7 is a bottom view of the patient treatment system of Fig. 1.

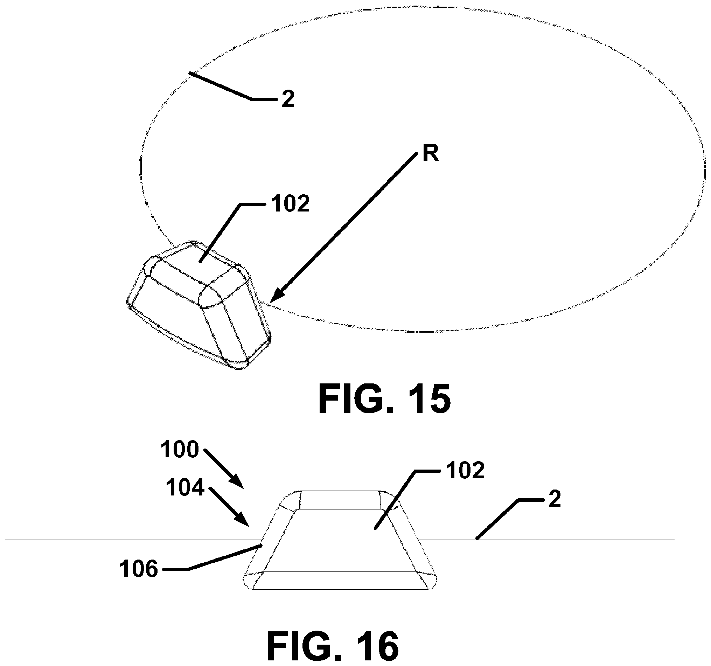

A patient treatment system comprises a cervical collar 10, one or more aids 100 (shown by example in Figs. 15-18, 19a, 19b, 21a, 21b, and/or 23a-23c) one or more retainers 120 for retaining the one or more aids, and a closure device 140. Cervical collar 10 supports and/or immobilizes a patient's neck by preventing unintended movement of the neck vertebrate. Cervical collar and/or a patient's head by limiting the movement of a patient's chin and/or lower jaw.

An aid may be an aid 100, which may be a temperature regulating aid and/or any other aid that provides comfort to a patient regardless of the particular size of the patient's neck or contour of the neck by maintaining aid 100 adjacent to skin of the patient's neck.

While the patient treatment system in this application is described with respect to an adult human patient, the patient treatment system may be altered and used with veterinary, pediatric, or geriatric patient by making changes to sizes, dimensions, and proportions of the components of the patient treatment system as may be exercised by one having ordinary skill in the art. The word "patient" should not be construed in a strictly medical sense. "Patient" may refer to a person that is not under medical care. For example, the present invention may be used by a race car driver who because of the inherent danger of racing is required to wear a cervical collar and may wish to have one or more temperature regulating aids 100 in order to maintain optimal body temperature for increased concentration.

Cervical collar 10 comprises a substrate 20 and a padding 40 that is connected via one or more connectors 32 to the substrate. Substrate 20 is preferably made of a first material 22 that is flexible yet maintains a shape imparted to it when manufactured and provides a support to a patient's neck. Preferably, substrate 20 is manufactured using injection molding and material 20, preferably, comprises one or more thermoplastics. In the alternative, substrate 20 may be made by stamping it from a sheet of material 22 that, preferably, comprises a plastic, thermoplastic, and/or a thermoplastic and elastomer mix. However, material 22 may be any other suitable material or combination of materials known or yet to be developed that is or are flexible yet maintain a shape imparted to it when manufactured as well as be easy to clean and/or sterilize by means known in the art.

Padding 40 comprises a structure having a single layer or a plurality of layers comprising at one material 42 that provides a cushioning effect between the patient's neck and substrate 20. Preferably, material 42 is substantially less stiff than material 22 and may comprise a foam material, a rubberized foam, a gel material, and/or any other suitable material.

Padding 40 may also include an outer surface layer 42 that is easy to clean, resists staining, and/or provides a low friction contact to the patient's neck to minimize patient discomfort as the patient's neck moves relative to cervical collar 10 and/or padding 40. In certain circumstances, it would be preferable to have padding 40 be made inexpensively to permit the padding to be disposable. In other circumstances, padding 40 may be reusable.

Cervical collar 10 comprises a plurality of portions: a forward portion 12 and a rearward portion 14 joined by a hinge 16. Each of substrate 20 and padding 40 are disposed in each of the portions, such that the substrate comprises respective substrate forward portion 12a, substrate rearward portion 14a, and substrate hinge portion 16a and the padding comprises respective padding forward portion 12b, padding rearward portion 14b, and padding hinge portion 16b.

When cervical collar 10 is used by the patient, forward portion 12, i.e., portions 12a and 12b, covers the front portion of the patient's neck and a lateral portion, preferably, up to and including the trapezius muscles on each side of the patient's neck. The rearward portion 14, i.e., portions 14a and 14b, cover the rear portion of the neck with the hinge 16, i.e., portions 16a and 16b covering and gap in between.

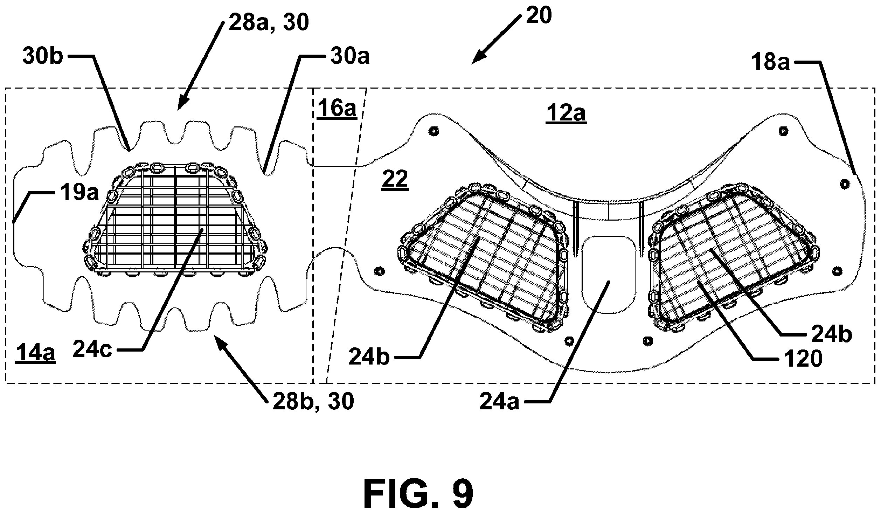

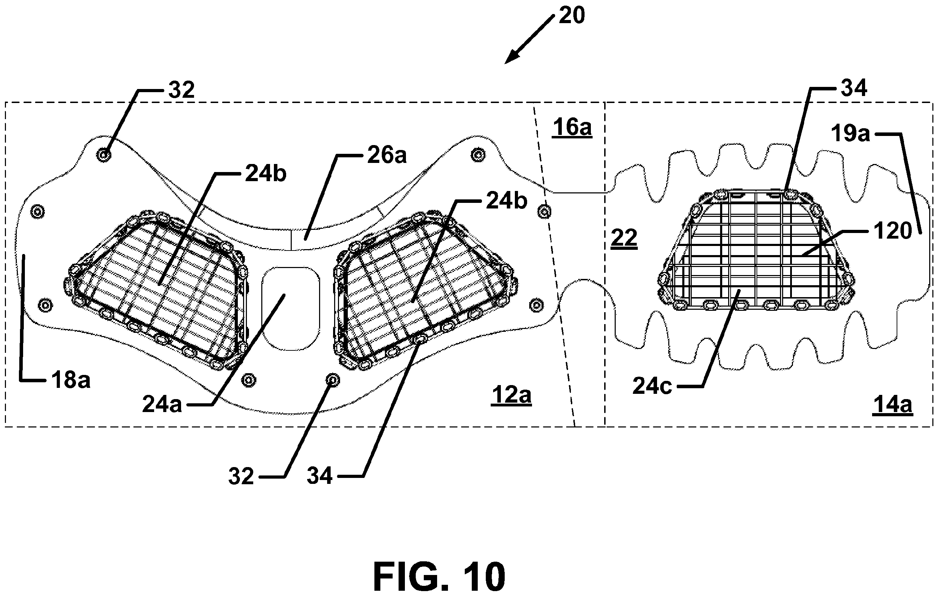

Closure device 140 then secures a distal end 18, i.e., substrate distal end 18a (shown in Figs. 9 and 10), to a proximate end 19, i.e., substrate proximal end 19a (shown in Figs. 9 and 10), to close cervical collar 10 into an annular shape about the patient's neck using any suitable closure means. However, preferably, a hook-and-loop fastener system is used.

In keeping with the general purpose of cervical collar 10 lacks sharp edges and has rounded corners to minimize injury due to the proximity of the cervical collar to the patient.

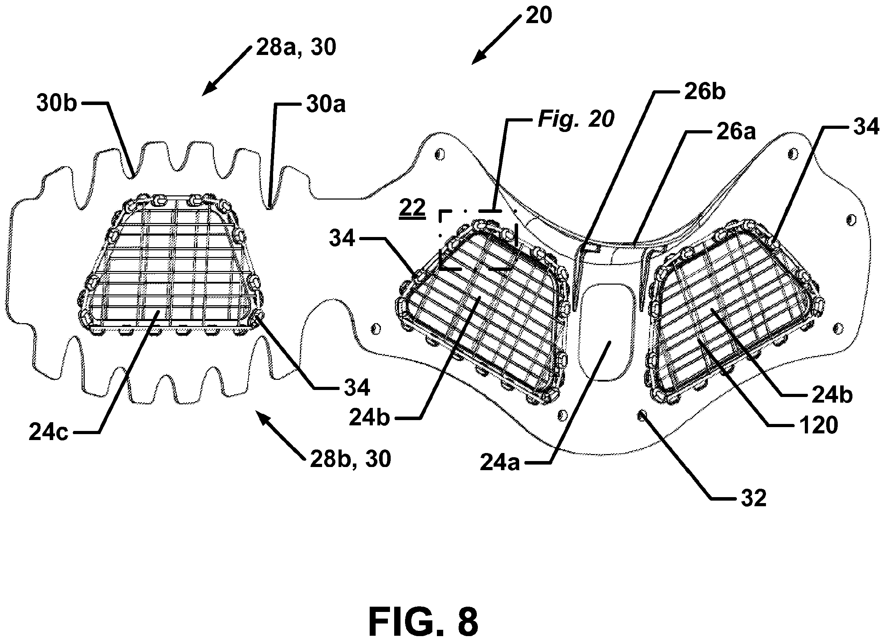

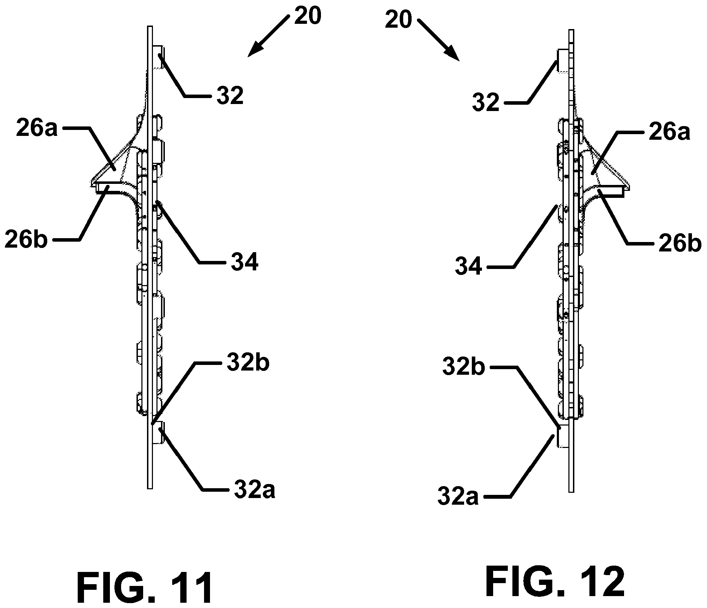

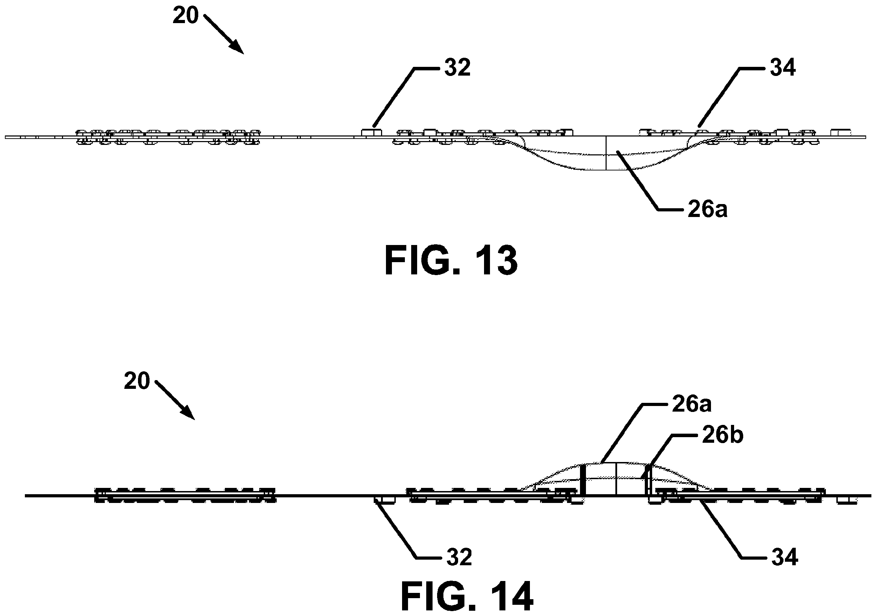

Fig. 8 is a perspective view of a substrate of the patient treatment system of Fig. 1 in accordance with one or more embodiments of the present invention. Fig. 9 is a front view of the substrate of Fig. 8. Fig. 10 is a rear view of the substrate of Fig. 8. Fig. 11 is a left-side view of the substrate of Fig. 8. Fig. 12 is a right-side view of the substrate of Fig. 8. Fig. 13 is a top view of the substrate of Fig. 8. Fig. 14 is a top view of the substrate of Fig. 8.

Substrate forward portion 12a comprises a general bi-lateral V shape having one or more central openings 24a and one or more lateral openings 24b. Each central opening 24a has a general rectangular shape and each lateral opening 24b has a trapezoidal shape. Preferably, only one central opening 24a is present so that the medical professional assisting the patient has an obstructed access to the trachea and surrounding area of the front of the throat to perform, for example, a tracheotomy, or another medical procedure. Thus, substrate 20 is strong enough that a perimeter portion surrounding central opening 24a maintains the opening with collapsing.

Similarly, each lateral opening 24b is sized so that one or more aids 100 may be disposed in the opening. Preferably, each lateral opening 24b is sized large enough that a single aid may be disposed in the opening and covers the entire lateral side of the patient's neck. Thus, substrate 20 is strong enough that the perimeter portions surrounding lateral openings 24b maintain the openings with collapsing.

Substrate forward portion 12a comprises a chin support 26a that extends substantially perpendicular to a plane passing through substrate forward portion 12a and that includes openings 24a and 24b. One or more support brackets 26b provide structural support to chin support 26a.

Substrate rearward portion 14a comprises a rear neck opening 24c configured substantially identical to lateral openings 24b that places an aid directly on the brain stem of the patient. Substrate rearward portion 14a also comprises edge portions 28a and 28b, each having a plurality of indents 30 that permit substrate rearward portion 14 to bend around the rear of the patient's neck. Edge portions 28a and 28b are preferably disposed on opposed sides and each portion preferably includes a pair of deep indents 30a and a plurality of shallower indents 30b. Therein, the indents of edge portions 28a and 28b comprise centerlines that are in line with each other for a more ergonomic fit as it curves around the rear of the patient's neck.

A rear side of substrate 20 comprises one or more connectors 32 that aid in securing padding 40 to substrate 20 and/or inhibiting the unintended removal of padding 40 from substrate 20. One or more connectors comprises at least a retaining portion 32a and an extending portion 32b that extends from a surface of substrate 20 to retaining portion 32a. Extending portion 32b may have an annular shape in cross-section and may taper, in an elevation view, from wide to narrow from base at the surface of the substrate to a portion adjacent and/or connected directly the retaining portion.

Retaining portion 32a preferably has an edge that extends over or is cantilevered over the portion of extended portion 32b that is adjacent to the retaining portion. However, one or more connectors 32 may also comprise a hook shape or other means for securing padding 40 to substrate 20 and/or inhibiting the unintended removal of padding 40 from substrate 20. Therein, connector 32 may be press-formed, i.e., pressed out, from substrate 20, may be molded integrally with substrate 20, or may be glued, sonically welded or in some other way joined or adjoined to substrate 20.

A plurality of connectors 34 is formed proximal to the edge of each lateral opening 24b and 24c on both the front side and the rear side of substrate 20. One or more connectors 34 are formed to have an edge 34a that extends over an upright portion 34b. Upright portion 34b is used to capture a portion of a peripheral edge 126 of retainer 120 while edge 34a prevents the unintended removal of the retainer 120 by averting the captured portion of the peripheral edge from sliding off the upright portion 34b.

Padding 40 preferably comprises openings 44a, 44b, and 44c that respectively match openings 24a, 24b, and 24c. However, openings 44a, 44b, and 44c are slightly larger to accommodate connectors 34 and the necessary space to efficiently remove and/or install retainers 120.

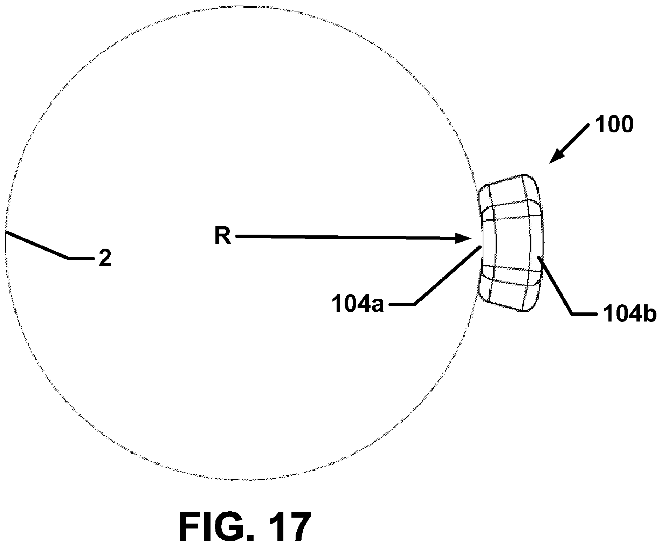

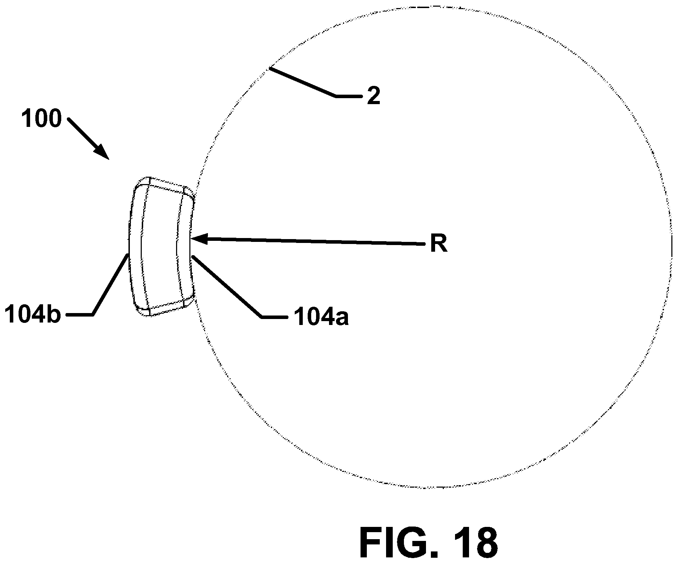

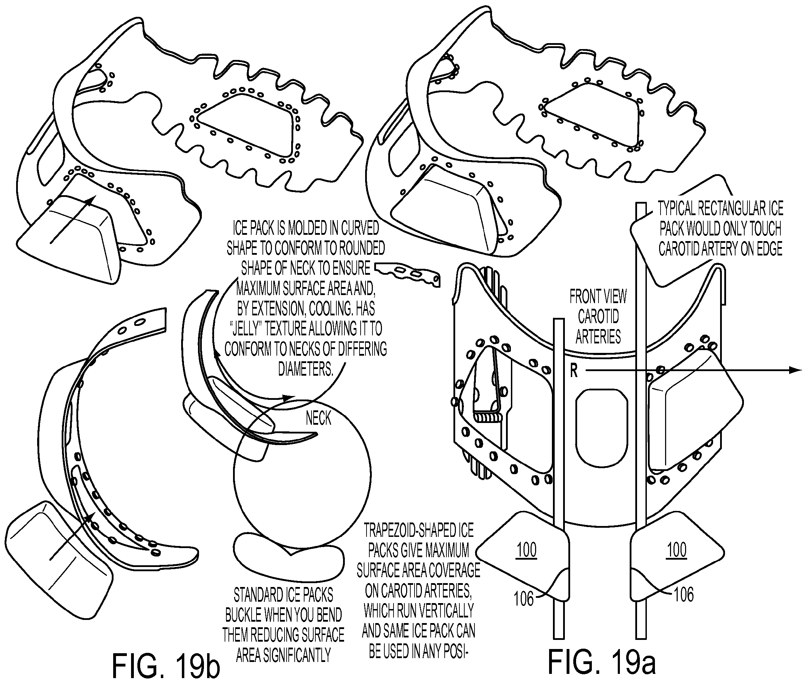

Fig. 15 is a perspective view of an aid for a patient treatment system of Fig. 1 in accordance with one or more embodiments of the present invention. Fig. 16 is a front view of the aid of Fig. 15. Fig. 17 is a bottom view of the aid of Fig. 15. Fig. 18 is a top view of the aid of Fig. 15. Fig. 19a is a schematic illustrating the positional advantage of the aid in accordance with one or more embodiments of the present invention. Fig. 19b is a schematic contrasting the advantages of the prior art and the advantages of the present invention.

Aid 100 may comprise any suitable aid that provides comfort to a patient. Thus, aid 100 may be a temperature regulating aid that heats or cools the patient's brain and/or body. Therein, at least one aid 100 is preferably placed in each of the lateral openings 24b and on the skin of the patient proximal to the carotid arteries, i.e., the external and internal carotid arteries, and/or placed on the skin of the patient proximal to the carotid body to heat or cool the oxygenated blood entering the brain.

Indeed, as is generally known in medicine the carotid body functions as a sensor by responding, inter alia, oxygen levels in the blood for proper central nervous system functioning. Since the carotid body is very sensitive to temperature in performing its sensory functioning, being able to affect the temperature to return a patient to medically-indicated preferred levels. Aid 100 placed in one of the lateral openings 24b may also control the body temperature by being placed on the skin of the patient proximal to the jugular vein. An aid 100 placed in rear opening 24c advantageously cools the brain stem directly. When aids 100 are placed in all three openings 24b and 24c, efficient and effective cooling of the brain is possible.

Aid 100 may also other aid that provides comfort or treatment to a patient. For example, aid 100 may comprise, in addition or instead of a temperature regulating device, and a pulse oximetry sensor to determine oxygen saturation levels, blood pressure monitor, a combination of the two, and/or any other suitable instruments.

Aid 100 comprises any suitable material. Preferably, when aid 100 is a temperature regulating device that cools, aid 100 comprises an outer skin 102 forming a closed container. Aid 100 then includes a gel material or any other suitable material that becomes cool or cold when exposed to lower temperatures. Therein, aid 100 does not need to be maintained except for a cleaning of the outer surface.

Preferably, when aid 100 is a temperature regulating device that heats, aid 100 comprises an outer skin that diffuses heat and does not create localized hot spots that can injure a patient. Aid 100 may contain any suitable material that becomes warm or hot when exposed to higher temperatures. Aid 100 may also include a heating device such as one operated by batteries or any other energy source.

Aid 100 comprises a general shape 104 that fits comfortably within lateral openings 24b and/or rear neck opening 24c and thus, preferably has in a planar view a general trapezoidal shape that is slightly smaller than lateral openings 24b, 24c by 1-5 mm on each side. In accordance with one or more embodiments of the present invention, one or more aids 100 may be also suitably sized to fit within central opening 24a.

As illustrated in Fig. 19a trapezoidal shaped aids 100 when placed into respective lateral openings 24b comprise an edge 106 that advantageously places the edges 106 directly on the carotid arteries. A typical rectangular shape would only touch the carotid artery on a corner.

Aid 100 comprises a base having a first surface 104a that is curved approximately to the curve of a patient's neck. Thus, the base advantageously provides maximum exposure to aid 100 to efficiently and quickly transfer cooling or heat to the patient. Surface 104a is placed against or adjacent to the patient's skin. A second surface 104b is spaced from surface 104a and preferably also is curved for the comfort of the patient and to provide a smooth surface. A plurality of edge surfaces 102c connects surfaces 104a and 104b. Surface 104a preferably has a radius R approximating the size of a patient's neck. Therein, radius R may be any radius that is suitable for various sized cervical collars 10, such as adult male, adult female, pediatric, infants or even animals.

In particular, outer skin 102 is preferably formed of pliable, elastic and/or flexible material that maintains close contact with the neck of the patient. For example, outer skin 102 may comprise a silicon material or a plastic material that is pliable, elastic and/or flexible and takes on the contours of the patient's neck but also maintain the curved shape of surface 104a. A standard ice pack is too flexible and buckles when placed against the neck as illustrated in Fig. 19b.

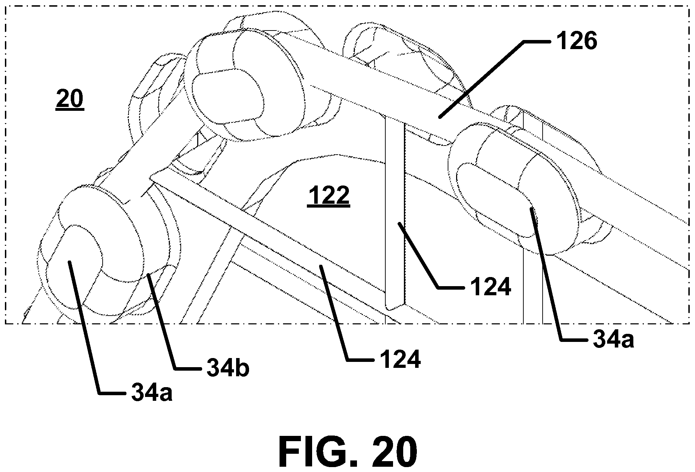

Fig. 20 is a detailed view of a retainer in accordance with one or more embodiments of the present invention. Retainer 120 may be any suitable material that preferably is stretchable and yet is able to contain an aid or aids 100 within respective opening or openings 24a, 24b and/or 24c. Thus, retainers 120 may be stretch cord, plastic cord, and/or other material arranged in a netting having a plurality of openings 122 formed by support members 124 and having a peripheral edge 126.

Retainers 120 may be placed in pairs, one on each side of the substrate and are secured via connectors 34. Aid 100 is held between the retainers and easily exchanged when necessary, such as when aid 100 is losing its cooling effect, by removing one of the retainers and adding a replacement aid 100. Advantageously, aid 100 is able to be maintained adjacent to the patient's skin regardless of the particular size of the patient's neck or contour of the neck by retainers 120 and, also by the use of pliable, elastic and/or flexible materials as the material choice for outer skin 102 of aid 100.

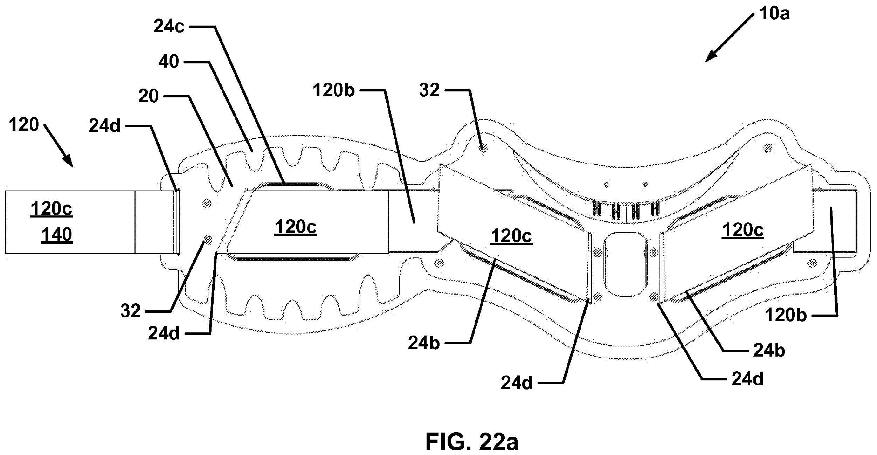

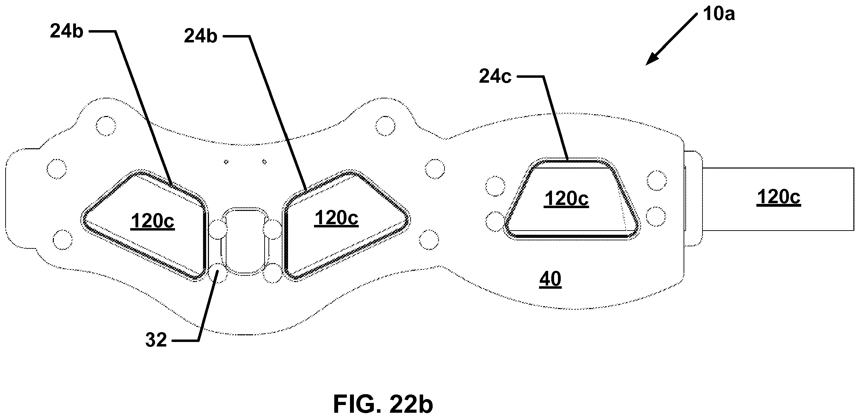

Fig. 22a is a front view of a patient treatment system in accordance with one or more embodiments of the present invention. Fig. 22b is a rear view of the patient treatment system of Fig. 22a.

Therein, the patient treatment system is configured substantially similar to embodiments of patient treatment systems disclosed above and, as further disclosed herein. The patient treatment system comprises a cervical collar 10a, which is configured substantially similar to collar 10, one or more aids (shown in Figs 15-18, 19a, 19b, 21a, 21b, and 23a-23c), one or more retainers 120a for retaining the one or more aids, and a closure device 140 and having openings 24ba and 24c wherein the aids are disposed. Cervical collar 10a comprises a substrate 20 and a padding 40 that is connected via one or more connectors 32 to the substrate.

While collar 10 uses retainers 120 that may be arranged in a netting that is at least partially secured in connectors 34 surrounding openings 24b and 24c, collar 10a uses one or more retainers 120. Each retainer 120 comprises first member 120b and a second member 120c that secure to each other using a hook-and-loop fastener assembly, such as Velcro™. One portion of the hook-and-loop fastener is disposed on the first member and another portion of the hook-and-loop fastener is disposed on the second member.

The first member is preferably secured to substrate 20 by, for example, being glued, sonically welded, or the like and is disposed between the substrate and the padding. Thus, first member 120b remains static with respect to substrate 20. The first member may be made of a substantially non-stretchable base onto which the one portion of the hook-and-loop fastener is disposed or, if the first member consists of the one portion of the hook-and-loop fastener, the one portion of the hook-and-loop fastener is preferably non-stretchable.

The second member may be a strap, band, or ribbon of stretchable or non-stretchable material that extends over one of the respective openings 24b or 24c and retain a respective aid in the opening when the second member is secured to the first member. Second member 120c pass through slit openings 24d and are secured to substrate 20, i.e., between the substrate and the padding by sandwiching the retainer, with one or more connectors 32.

In use, an aid is placed in openings 24b and/or 24c, the aid is activated (as explained below) and second member 120c is then pulled over the aid and secured to first member 120b to retain the aid in respective openings 24b and/or 24c. In other words, retainer 120 of Fig. 10a has a first end (at the static end of member 120b) and a second end (at the free end of member 120c). The first end is disposed between the substrate and the padding. Moreover, collar 10a may also include a closure device such as device 140.

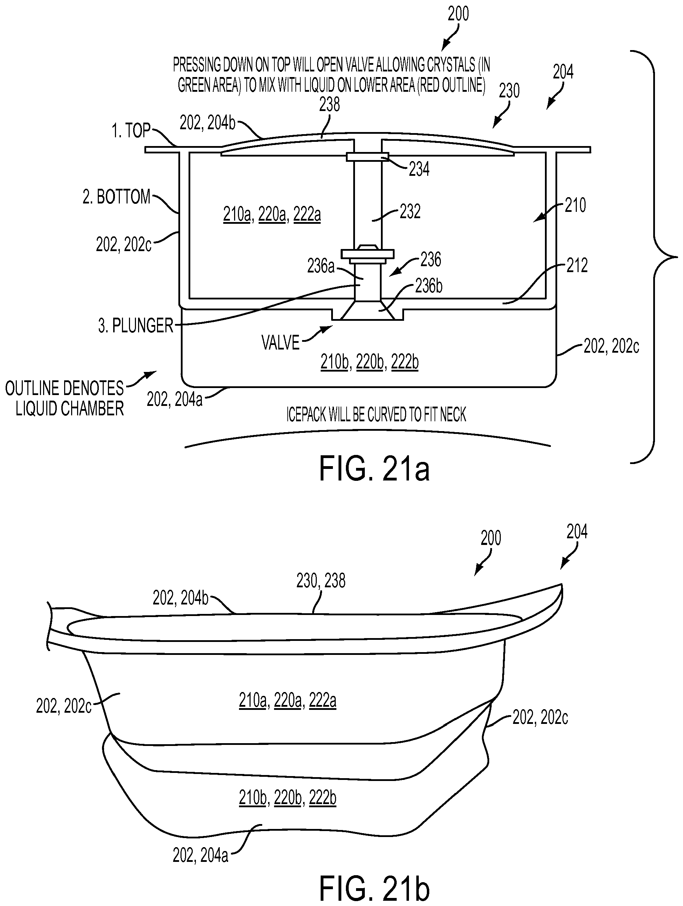

Fig. 21a is a cross-sectional view of an aid for a patient treatment system in accordance with one embodiment of the present invention. Fig. 21b is an isometric view of an aid for a patient treatment system in accordance with one embodiment of the present invention.

An aid 200 is configured as a temperature regulating aid that heats or cools the patient's brain and/or body. Aid 200 may selectively incorporate one or more features of aid 100 or may include all features of aid 100 and may be used in the same way as aid 100. Therein, at least one aid 200 is preferably placed in each of the lateral openings 24b and on the skin of the patient proximal to the carotid arteries, i.e., the external and internal carotid arteries, and/or placed on the skin of the patient proximal to the carotid body to heat or cool the oxygenated blood entering the brain.

However, aid 200 preferably utilizes a chemical reaction of one or more chemicals, substances, and/or compounds, i.e., "reacting agents," to achieve heating or cooling. In accordance with one or more embodiments of the present invention, aid 200 cools by releasing one or more first reacting agents 220a to mix with one or more second reacting agents 220b to create an endothermic chemical reaction. In accordance with one or more embodiments of the present invention, aid 200 cools by releasing one or more first reacting agents 222a to mix with one or more second reacting agents 222b to create an exothermic chemical reaction.

The one or more first reacting agents and the one or more second reacting agents are preferably chosen so that the chemical reaction among them creates a "thermic reaction", i.e., endothermic or exothermic reaction, that is "suitable for a medical patient" in a health treatment setting such as a hospital, emergency services ambulance, stretcher, nursing home, and/or a patient's home. "Suitable for a medical patient" means that the thermic reaction does not irreversible damage the skin, organs, nerves, brain, and/or other body parts of the patients and/or irreversibly limits the functions of any body part of the patient.

In accordance with one or more embodiments of the present invention, an endothermic chemical reaction is achieved
first reacting agents 222a (as a liquid form) comprise or consist of (as measured by volume):
   Water: 99.890244%
   Propylene Glycol: 0.104878%
   Flavonoid: 0.004878%
   and
second reacting agents 222b (in a dry form) comprise or consist of (as measured by volume):
   Hydroxyproline: 40.000%
   Alginic Acid Sodium Salt: 30.000%
   Betaine: 30.000%.

Therein, only reacting agents 222a and 222b are used in the endothermic chemical reaction.

Indeed, as is generally known in medicine the carotid body functions as a sensor by responding, inter alia, oxygen levels in the blood for proper central nervous system functioning. Since the carotid body is very sensitive to temperature in performing its sensory functioning, being able to affect the temperature to return a patient to medically-indicated preferred levels. Aid 200 placed in one of the lateral openings 24b (Figs. 1 and 10) may also control the body temperature by being placed on the skin of the patient proximal to the jugular vein. An aid 200 placed in rear opening 24c (Figs. 1 and 10) advantageously cools the brain stem directly. When aids 200 are placed in all three openings 24b and 24c, efficient and effective cooling of the brain is possible.

Aid 200 may also other aid that provides comfort or treatment to a patient. For example, aid 200 may comprise, in addition to a temperature regulating device, and a pulse oximetry sensor to determine oxygen saturation levels, blood pressure monitor, a combination of the two, and/or any other suitable instruments.

Aid 200 comprises any suitable material and, preferably, comprises outer skin 202 that diffuses heat and/or cold and does not create localized hot and cold spots that can injure a patient. Aid 200 comprises a general shape 204 that fits comfortably within lateral openings 24b and/or rear neck opening 24c and thus, preferably has in a planar view a general trapezoidal shape that is slightly smaller than lateral openings 24b, 24c by 1-5 mm on each side. In accordance with one or more embodiments of the present invention, one or more aids 200 may be also suitably sized to fit within central opening 24a.

As illustrated in Fig. 15-18 and 19a with respect to aid 100, but also applicable to aid 200, trapezoidal shaped aids 200 when placed into respective lateral openings 24b comprise an edge, similar to edge 106, that advantageously places the edges, similarly to edges 106, directly on the carotid arteries. A typical rectangular shape would only touch the carotid artery on a corner.

Aid 200 comprises a base having a first surface 204a that is curved approximately to the curve of a patient's neck. Thus, the base advantageously provides maximum exposure to aid 200 to efficiently and quickly transfer cooling or heat to the patient. Surface 204a is placed against or adjacent to the patient's skin. A second surface 204b is spaced from surface 204a and preferably also is curved for the comfort of the patient and to provide a smooth surface. A plurality of edge surfaces 202c connects surfaces 204a and 204b. Surface 204a preferably has a radius R approximating the size of a patient's neck. Therein, radius R may be any radius that is suitable for various sized cervical collars 10 and/or 10a, such as adult male, adult female, pediatric, infants or even animals.

In particular, outer skin 202 is preferably formed of pliable, elastic and/or flexible material that maintains close contact with the neck of the patient. For example, outer skin 202 may comprise a silicon material or a plastic material that is pliable, elastic and/or flexible and takes on the contours of the patient's neck but also maintain the curved shape of surface 204a. A standard ice pack is too flexible and buckles when placed against the neck as illustrated in Fig. 19b with respect to aid 100.

Preferably, outer skin 202 forms a substantially closed container having an inner space 210 divided into at least a first space 210a for retaining the one or more first reacting agents 220a, 222a and a second space 210b for retaining the one or more second reacting agents 220b, 222b. It is possible that more than the one or more first reacting agents and the one or more second reacting agents may be needed or desired to create, reinvigorate, extend, slow, decrease, and/or moderate the thermic reaction suitable for a medical patient. Thus, inner space 210 may be divided such that additional reacting agents are housed in aid 200.

Inner spaces 210a and 210b are separated by one or more barriers 212 that each is preferably impervious and physically and chemically non-reacting with respect to all reacting agents 220a and 220b. When one or more additional reacting agents, i.e., additional to reacting agents 210a and 210b, are present, one or more additional barriers 212, each of which is impervious and physically and chemically non-reacting with all reacting agents.

Aid 200 further comprises an actuator assembly 230 that brings, permits, and/or induces the reacting agents in contact with each other to initiate, cause, and/or prolong the thermic reaction.

In accordance with one or more embodiments of the present invention, actuator assembly 230 comprises a first opening in outer skin 202 and an opening in barrier 212 in which a stem 232 is mounted. A seal 234 is disposed in the outer skin to prevent respective inner space 210a or 210b from being exposed to ambient air and/or leaking one or more reacting agents. A valve assembly 236 includes a plunger 236a and an initiator 236b. Plunger 236a is connected to the stem and when pressed acts on initiator 236b via stem 232. Thus, in a first state, initiator 236b is disposed in an opening in barrier 212 and operatively seals inner spaces 210a and 210b, i.e., reacting agents 220a and 220b, from each other.. In a second state, the plunger, as acted on by the actuator, causes the initiator to move and unseal the opening permitting reacting agents 220a and 220b to mix and initiate, cause, and/or prolong the thermic reaction.

An actuator 238 is situated proximal to a surface on aid 200. The actuator is formed as a button or other human-engageable device and is mounted on or operatively connected to stem 232. When actuator 238 is, preferably, depressed, i.e., pressed closer to aid 200 to cause stem 232 to operatively push on plunger 236a and disengage initiator 236b from its position. This permits reacting agents 220a and 220b to mix and initiate, cause, and/or prolong the thermic reaction. Advantageously, as the actuator is released and stem 232 returns to its original position, plunger 236a remains in its depressed position and causing initiator 236b to remain in its depressed position. This avoids requiring medical personnel to unnecessarily continue to depress the actuator so that the reacting agents continue to mix.

In accordance with one or more embodiments of the present invention, initiator 236b may be in the shape of a disc valve or a disc stopper having a substantially round shape in plan view and flat in an elevation view. The edges may be angled. The combination of the one or more shape features provides an economical seal, i.e., flat shape does not take up much space, disc shape permits even distribution of forces, and angled edges ensure that there is positive seating even with high manufacturing tolerances.

In accordance with one or more related embodiments of the present invention, actuator 238 may be moved further away from aid 200 to cause stem 232 to operatively disengage with valve assembly 236 and permit reacting agents 220a and 220b to mix and initiate, cause, and/or prolong the thermic reaction.

In accordance with one or more further related embodiments of the present invention, actuator 238, preferably in the form of a pull tab, and/or a portion of stem 232 may be removed from aid 200 to cause stem 232 to operatively disengage valve assembly 236 and permit reacting agents 220a and 220b to mix and initiate, cause, and/or prolong the thermic reaction.

In accordance with one or more further related embodiments of the present invention, a plurality of actuators 238 are present and selectively act on a respective plurality of stems 232 to operatively disengage a plurality of valve assembly 236 and permit a plurality reacting agents 220a and 220b to mix and initiate, cause, and/or prolong the thermic reaction.

In accordance with one or more embodiments of the present invention, actuator assembly 230 comprises a first opening in outer skin 202 and lacks any opening in barrier 212 in which a stem 232 is mounted. A seal 234 is disposed in the outer skin to prevent respective inner space 210a or 210b from being exposed to ambient air and/or leaking one or more reacting agents. The stem comprises a puncture tool capable of puncturing barrier 212 permit reacting agents 220a and 220b to mix and initiate, cause, and/or prolong the thermic reaction.

An actuator 238 is situated proximal to a surface on aid 200. The actuator is formed as a button or other human-engageable device and is mounted on or operatively connected to stem 232. When actuator 238 is, preferably, depressed, i.e., pressed closer to aid 200, to cause the puncture tool of stem 232 to puncture barrier 212 and permit reacting agents 220a and 220b to mix and initiate, cause, and/or prolong the thermic reaction.

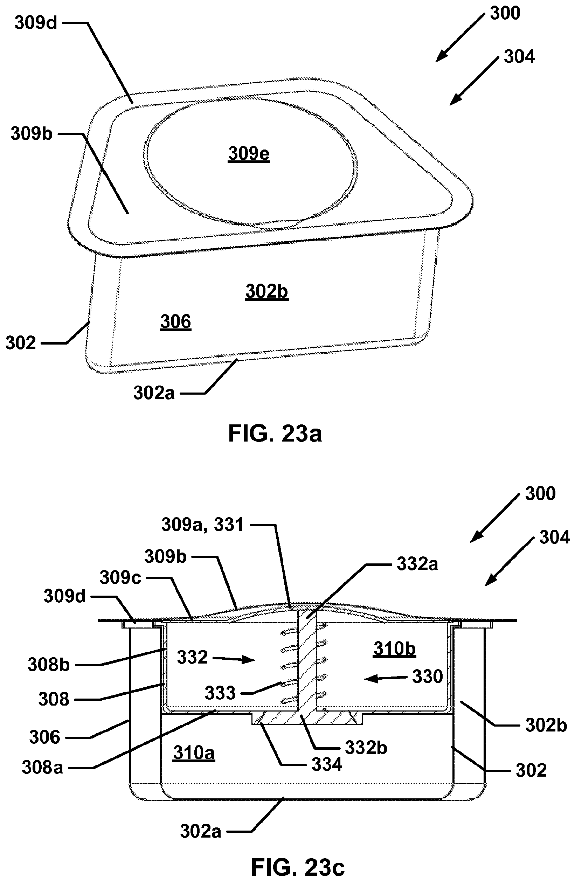

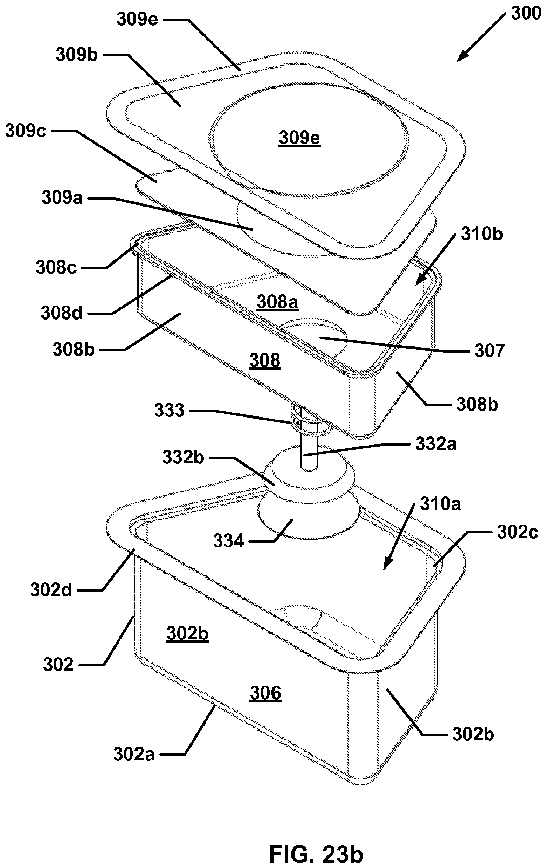

Fig. 23a is a perspective view of an aid for a patient treatment system in accordance with one embodiment of the present invention. Fig. 23b is an exploded view of the aid of Fig. 23a. Fig. 23c is a cross-sectional view of the aid of Fig. 23a.

An aid 300 is configured as a temperature regulating aid that heats or cools the patient's brain and/or body. Aid 300 may selectively incorporate one or more features of aid 100 and/or 200 or may include all features of aid 100 and/or 200 and may be used in the same way as aid 100 and/or 200. Therein, at least one aid 300 is preferably placed in each of the lateral openings 24b and on the skin of the patient proximal to the carotid arteries, i.e., the external and internal carotid arteries, and/or placed on the skin of the patient proximal to the carotid body to heat or cool the oxygenated blood entering the brain.

However, aid 300 preferably utilizes a chemical reaction of one or more chemicals, substances, and/or compounds, i.e., "reacting agents," to achieve heating or cooling. In accordance with one or more embodiments of the present invention, aid 300 cools by releasing one or more first reacting agents (not shown) to mix with one or more second reacting agents (not shown) to create an endothermic chemical reaction. In accordance with one or more embodiments of the present invention, aid 200 cools by releasing one or more first reacting agents (not shown) to mix with one or more second reacting agents (not shown) to create an exothermic chemical reaction.

The one or more first reacting agents and the one or more second reacting agents are preferably chosen so that the chemical reaction among them creates a "thermic reaction", i.e., endothermic or exothermic reaction, that is "suitable for a medical patient" in a health treatment setting such as a hospital, emergency services ambulance, stretcher, nursing home, and/or a patient's home. "Suitable for a medical patient" means that the thermic reaction does not irreversible damage the skin, organs, nerves, brain, and/or other body parts of the patients and/or irreversibly limits the functions of any body part of the patient.

In accordance with one or more embodiments of the present invention, an endothermic chemical reaction is achieved
first reacting agents (as a liquid form) comprise or consist of (as measured by volume):
   Water: 99.890244%
   Propylene Glycol: 0.104878%
   Flavonoid: 0.004878%
   and
second reacting agents (in a dry form) comprise or consist of (as measured by volume):
   Hydroxyproline: 40.000%
   Alginic Acid Sodium Salt: 30.000%
   Betaine: 30.000%.

Therein, only the first and second reacting agents are used in the endothermic chemical reaction. Advantageously, none of the ingredients of either or both reacting agents are poisonous, toxic, or injurious to a patient. Should in the midst of a treatment, an aid be punctured and leak on a patient, the patient is not in a danger or is further being hurt.

Aid 300 may also other aid that provides comfort or treatment to a patient. For example, aid 300 may comprise, in addition to a temperature regulating device, and a pulse oximetry sensor to determine oxygen saturation levels, blood pressure monitor, a combination of the two, and/or any other suitable instruments.

Aid 300 comprises any suitable material and, preferably, comprises outer skin 302 that diffuses heat and/or cold and does not create localized hot and cold spots that can injure a patient. Aid 300 comprises a general shape 304 that fits comfortably within lateral openings 24b and/or rear neck opening 24c and thus, preferably has in a planar view a general trapezoidal shape that is slightly smaller than lateral openings 24b, 24c by 1-5 mm on each side. In accordance with one or more embodiments of the present invention, one or more aids 300 may be also suitably sized to fit within central opening 24a.

As illustrated in Fig. 15-18 and 19a with respect to aid 100, but also applicable to aid 300, trapezoidal shaped aids 300 when placed into respective lateral openings 24b comprise an edge, similar to edge 106, that advantageously places the edges, similarly to edges 106, directly on the carotid arteries. A typical rectangular shape would only touch the carotid artery on a corner.

Aid 300 comprises an outer skin 302 and a base 302a having a surface that is curved approximately to the curve of a patient's neck. Thus, advantageously the base provides maximum exposure to aid 200 to efficiently and quickly transfer cooling or heat to the patient. Base 302a is placed against or adjacent to the patient's skin. A plurality of upright walls 302b connects to base 302a. Base 302a preferably has a radius R approximating the size of a patient's neck. Therein, radius R may be any radius that is suitable for various sized cervical collars 10 and/or 10a, such as adult male, adult female, pediatric, infants or even animals.

In particular, outer skin 302 is preferably formed of pliable, elastic and/or flexible material that maintains close contact with the neck of the patient. For example, outer skin 302 may comprise a silicon material or a plastic material that is pliable, elastic and/or flexible and takes on the contours of the patient's neck but also maintain the curved shape of base 302. A standard ice pack is too flexible and buckles when placed against the neck as illustrated in Fig. 19b with respect to aid 100.

Preferably, outer skin 302 forms a substantially closed first container 306 having an inner space 310a either the second reacting agent in, preferably a dry form. Therein, first container 306 comprises base 302a and upright walls 302b. Walls 302b terminate at preferably a peripherally located inner ledge 302c. An outer ledge 302d is stepped up and away from ledge 302c.

A second container 308 comprises a shape that fits entirely inside first container 306 and defines an inner space 310b. The second container comprises and opening 307 in a base 308a and upright walls 308b. Walls 308b terminate at preferably a peripherally located inner ledge 308c. An outer ledge 308d is stepped up and away from ledge 308c. Ledge 308c is received on ledge 302c such that ledge 302c supports container 308 leaving a space between the base of the second container and the base of the first container.

An inner sealing member 309a is received on ledge 308c to seal the second container to form a seal that prevents the unintended release of the first reacting agents. The inner sealing member preferably has a thickness such that sealing member 309a and ledge 308d are flush with the top surface of outside ledge 302d. An outer sealing member 309b is provided and seals the first container when joined to ledge 302d to form a seal that prevents the unintended release of the contents of aid 300. The outer sealing member may have an edge 309d that is glued, sonically welded, or the like to ledge 302d. The inner sealing member preferably is a solid, semi-flexible member having a raised portion in the center in direct contact with a portion of the actuator assembly.

Aid 300 further comprises an actuator assembly 330 that brings, permits, and/or induces the reacting agents in contact with each other to initiate, cause, and/or prolong the thermic reaction.

In accordance with one or more embodiments of the present invention, actuator assembly 330 comprises an actuator 331, a plunger 332 having a stem 332a and an initiator 332b, biasing member 333, preferably in the form of a spring, and a barrier seal 334. Barrier seal 334 keeps the initiator secured to the base of the second container to prevent mixing of the reacting agents. The barrier seal covers the entirety of opening 307 and a peripheral edge of the base around the opening. The initiator is disposed in opening 307, and therein, the spring prevents the initiator from returning into the opening.

In accordance with one or more embodiments of the present invention, initiator 332b may be in the shape of a disc valve or a disc stopper having a substantially round shape in plan view and flat in an elevation view. The edges may be angled. The combination of the one or more shape features provides an economical seal in opening 308, i.e., flat shape does not take up much space, disc shape permits even distribution of forces, and angled edges ensure that there is positive seating even with high manufacturing tolerances.

Actuator 331 is situated proximal to a surface on aid 300. The actuator is formed as a button or other human-engageable device such as the raised area in seal 309a and is mounted on or operatively connected to stem 332a. When actuator 331 is, preferably, depressed, i.e., pressed closer to aid 300, to cause the initiator 332b to move out of opening 307 and into the gap between the base of the second container and the base of the first container. This causes barrier seal 334 also to be broken or moved and also permits the first reacting agents, which are in liquid form, to leave the second container by the force of gravity and to mix with the second reacting agents, which are in dry form, and initiate, cause, and/or prolong the thermic reaction.

A top surface of outer seal 309b may be marked in some manner, such as indicating thumbs markings to provide ready visual indication to a user to depress the actuator. A portion 309e of the outer seal may be removable, such as being peelable in order to access the actuator.

In accordance with one or more embodiments of the present invention, aid 100 may also be configured to be an intravenous delivery device that comprises one or more pharmaceuticals, nutrients, plasma, blood, or other medically necessary material stored in a reservoir. Therein, the aid may comprise an actuator operatively releasing and/or advancing a needle into the carotid artery of the patient. The needle is connected to the reservoir such that the contents of the reservoir may be medically delivered to the patient.

In accordance with one or more embodiments of the present invention, aid 100 may comprise a GPS unit, a Wi-Fi unit, or location-based sensing unit to track the location of a patient using the patient treatment system.

In accordance with one or more embodiments of the present invention, aid 100 may comprise a data recorder for recording the condition of a patient using the patient treatment system and may record data over a sustained period of time.

In accordance with one or more embodiments of the present invention, aid 100 may comprise a video recorder to record a treatment record as being the patient is being treated by an EMT.

While the invention has been described in conjunction with specific embodiments, it is to be understood that many alternatives, modifications, and variations will be apparent to those skilled in the art in light of the foregoing description.

## Claims

1. A cervical collar (10) for a patient, the cervical collar (10) comprising:
a first opening (24a) located over a trachea of the patient;
the first opening (24a) being sized to permit a medical professional to have an unobstructed access to the trachea and surrounding area of a front of the throat to perform a tracheotomy,
**characterized in that**
a second opening (24b) located over a carotid artery of the patient is provided.

2. The cervical collar (10) of claim 1, further comprising
the first opening (24a) having a generally rectangular shape, and
the second opening (24b) having a trapezoidal shape.

3. The cervical collar (10) of claim 1, further comprising a rear opening (24c), the rear opening (24c) for proving access to a neck of the patient.

4. The cervical collar (10) of claim 1, further comprising a substrate (20), a padding (40), and a retainer (120); the retainer (120) having a first end and a second end, the first end disposed between the substrate (20) and the padding (40) and the second end having a portion of a hook-and-loop fastener.

5. The cervical collar (10) of claim 4, the substrate (20) comprising a forward portion (12a), the forward portion (12a) having a V-shape, the first opening (24a) being centrally located in the forward portion (12a) and the second opening (24b) being laterally located in the forward portion (12a).

6. The cervical collar (10) of claim 4, wherein the substrate (20) comprises a rearward portion (14a), the rearward portion (14a) comprising a rear opening (24c), the rear opening (24c) for providing access to a neck of the patient.

7. The cervical collar (10) of claim 1, further comprising a substrate (20), the substrate (20) comprising a forward portion (12a), a rearward portion (14a), and a hinge portion (16a), the hinge portion (16a) being disposed between the forward portion (12a) and the rearward portion (14a),
the first opening (24a) being centrally located in the forward portion (12a) and the second opening (24b) being laterally located in the forward portion (12a);
a rear opening (24c) in the rearward portion (14a), the rear opening (24c) for providing access to a neck of the patient.

8. The cervical collar (10) of claim 7, when the rearward portion (14a) comprises a plurality of indents (30) at an edge (28a, 28b) of the rearward portion (14a).

9. A patient treatment system comprising a cervical collar (10) according to claim 1 for treating a patient, the patient treatment system comprising:
an aid (200) disposed in the second opening (24b), the aid (200) comprising a base having a curved surface (204a), the base being disposed proximal to a neck of the patient over the carotid artery, the aid (200) comprising a first reacting agent (220a) and a second reacting agent (220b);
wherein the first reacting agent (220a) and the second reacting agent (220b) create a thermic reaction when mixed.

10. The patient treatment system of claim 9, wherein the aid (300) further comprises
a first container (306), the first container (306) including the base (302a);
a second container (308) disposed entirely within in the first container (306);
an actuator (331) pressed by a user;
an initiator (332b), in a first state, sealing the first reacting agent and the second reacting agent from each other, the initiator being responsive to the actuator to move to a second state permitting the first reacting agent and the second reacting agent to mix.

11. The patient treatment system of claim 10, wherein the first reacting agent is liquid and the second reacting agent is in dry form.

12. The patient treatment system of claim 11, wherein
the first reacting agent consists of
Water 99.890244% of the total volume of the first reacting agent,
Propylene Glycol 0.104878% of the total volume of the first reacting agent,
Flavonoid 0.004878% of the total volume of the first reacting agent, and and
the second reacting agent consists of
Hydroxyproline 40.000% of the total volume of the second reacting agent,
Alginic Acid Sodium Salt 30.000% of the total volume of the second reacting agent, and
Betaine 30.000% of the total volume of the second reacting agent.

13. The patient treatment system of claim 10, wherein the actuator (331) and the initiator (332b) are linked by a stem (332a).

14. The patient treatment system of claim 9, further comprising a retainer (120) to hold the aid in the second opening.

15. The patient treatment system of claim 14, further comprising a connector (32), a substrate (20), and a padding (40), the connector (32) sandwiching the retainer (120) between the substrate (20) and the padding (40).

## Patentansprüche

1. Zervikalstütze (10) für einen Patienten, wobei die Zervikalstütze (10) umfasst:
eine erste Öffnung (24a), die über einer Luftröhre des Patienten angeordnet ist;
wobei die erste Öffnung (24a) eine Abmessung aufweist, die es erlaubt, dass ein Berufsmediziner einen ungestörten Zugang zu der Luftröhre und zu umgebendem Gebiet einer Front der Kehle hat, um einen Luftröhrenschnitt durchzuführen,
**dadurch gekennzeichnet, dass**
eine zweite Öffnung (24b) über einer Halsschlagader des Patienten zur Verfügung gestellt wird.

2. Zervikalstütze (10) nach Anspruch 1, weiterhin umfassend,
dass die erste Öffnung (24a) eine im Wesentlichen rechteckige Gestalt aufweist und
dass die zweite Öffnung (24b) eine im Wesentlichen trapezförmige Gestalt aufweist.

3. Zervikalstütze (10) nach Anspruch 1, die weiterhin eine Rücköffnung (24c) umfasst, wobei die Rücköffnung (24c) einen Zugang zu einem Nacken des Patienten zur Verfügung stellt.

4. Zervikalstütze (10) nach Anspruch 1, die weiterhin ein Substrat (20), eine Polsterung (40) und einen Halter (120) umfasst; wobei der Halter (120) ein erstes Ende und ein zweites Ende aufweist, wobei das erste Ende zwischen dem Substrat (20) und der Polsterung (40) angeordnet ist und das zweite Ende einen Abschnitt eines Klettbefestigers aufweist.

5. Zervikalstütze (10) nach Anspruch 4, wobei das Substrat (20) einen Vorwärtsabschnitt (12a) umfasst, wobei der Vorwärtsabschnitt (12a) eine V-Form aufweist, wobei die erste Öffnung (24a) in dem Vorwärtsabschnitt (12a) zentral angeordnet ist und die zweite Öffnung (24b) in dem Vorwärtsabschnitt (12a) seitlich angeordnet ist.

6. Zervikalstütze (10) nach Anspruch 4, wobei das Substrat (20) einen Rückwärtsabschnitt (14a) aufweist, wobei der Rückwärtsabschnitt (14a) einen Rückwärtsöffnung (24c) umfasst, wobei die Rückwärtsöffnung (24c) zum Bereitstellen eines Zugangs zu einem Nacken des Patienten vorgesehen ist.

7. Zervikalstütze (10) nach Anspruch 1, die weiterhin ein Substrat (20) umfasst, wobei das Substrat (20) einen Vorwärtsabschnitt (12a), einen Rückwärtsabschnitt (14a) und einen Scharnierabschnitt (16a) umfasst, wobei der Scharnierabschnitt (16a) zwischen dem Vorwärtsabschnitt (12a) und dem Rückwärtsabschnitt (14a) angeordnet ist,
wobei die erste Öffnung (24a) zentral in dem Vorwärtsabschnitt (12a) angeordnet ist und die zweite Öffnung (24b) seitlich in dem Vorwärtsabschnitt (12a) angeordnet ist;
wobei eine Rückwärtsöffnung (24c) in dem Rückwärtsabschnitt (14a) vorgesehen ist, wobei die Rückwärtsöffnung (24c) zum Bereitstellen eines Zugangs zu einem Nacken des Patienten vorgesehen ist.

8. Zervikalstütze (10) nach Anspruch 7, bei der der Rückwärtsabschnitt (14a) eine Vielzahl von Kerben (30) an einer Kante (28a, 28b) des Rückwärtsabschnittes (14a) umfasst.

9. Patientenbehandlungssystem mit einer Zervikalstütze (10) nach Anspruch 1 zum Behandeln eines Patienten, wobei das Patientenbehandlungssystem umfasst:
eine Hilfseinrichtung (200), die in der zweiten Öffnung (24b) angeordnet ist, wobei die Hilfseinrichtung (200) eine Basis mit einer gekrümmten Oberfläche (204a) umfasst, wobei die Basis proximal bezügliche eines Nackens des Patienten über der Halsschlagader angeordnet ist, wobei die Hilfseinrichtung (200) ein erstes Reaktionsmittel (220a) und ein zweites Reaktionsmittel (220b) umfasst;
wobei das erste Reaktionsmittel (220a) und das zweite Reaktionsmittel (220b) einen thermische Reaktion erzeugen, wenn sie gemischt werden.

10. Patientenbehandlungssystem nach Anspruch 9, wobei die Hilfseinrichtung (300) weiterhin umfasst:
einen ersten Behälter (306), wobei der erste Behälter (306) eine Basis (302a) enthält;
einen zweiten Behälter (308), der vollständig innerhalb des ersten Behälters (306) angeordnet ist;
einen Aktuator (331), der von einem Nutzer gedrückt wird;
einen Initiator (332b), der in einem ersten Zustand das erste Reaktionsmittel und das zweite Reaktionsmittel voneinander trennt, wobei der Initiator auf den Aktuator reagiert, indem er in einen zweiten Zustand übergeht, in dem erlaubt wird, dass sich das erste Reaktionsmittel und das zweite Reaktionsmittel mischen.

11. Patientenbehandlungssystem nach Anspruch 10, wobei das erste Reaktionsmittel eine Flüssigkeit ist und das zweite Reaktionsmittel in trockener Form vorliegt.

12. Patientenbehandlungssystem nach Anspruch 11, wobei
das erste Reaktionsmittel zusammengesetzt ist aus
99,890244% Wasser bezüglich des Gesamtvolumens des ersten Reaktionsmittels,
0,104878% Propylenglykol bezüglich des Gesamtvolumens des ersten Reaktionsmittels,
0,004878% Flavonoid bezüglich des Gesamtvolumens des ersten Reaktionsmittels, und
wobei das zweite Reaktionsmittel zusammengesetzt ist aus
40,000% Hydroxyprolin bezüglich des Gesamtvolumens des zweiten Reaktionsmittels,
30,000% alginsaures Natriumsalz bezüglich des Gesamtvolumens des zweiten Reaktionsmittels und
30,000% Betain bezüglich des Gesamtvolumens des zweiten Reaktionsmittels.

13. Patientenbehandlungssystem nach Anspruch 10, wobei der Aktuator (331) und der Initiator (332b) durch eine Stange (332a) verbunden sind.

14. Patientenbehandlungssystem nach Anspruch 9, das weiterhin eine Halterung (120) zum Halten der Hilfseinrichtung in der zweiten Öffnung umfasst.

15. Patientenbehandlungssystem nach Anspruch 14, das weiterhin einen Verbinder (32), ein Substrat (20) und eine Polsterung (40) umfasst, wobei der Verbinder (32) die Halterung (120) sandwichartig zwischen dem Substrat (20) und der Polsterung (40) umgibt.

## Revendications

1. Le brevet porte sur un collier cervical (10) destiné aux patients, le collier cervical (10) comprenant :
une première ouverture (24a) située au-dessus de la trachée du patient,
la première ouverture (24a) étant dimensionnée pour permettre à un professionnel de santé d'accéder librement à la trachée et à la région avoisinante à l'avant de la gorge pour effectuer une trachéotomie,
**caractérisé en ce que**
une seconde ouverture (24b) est prévue au-dessus de l'artère carotide du patient.

2. Le collier cervical (10) d'après la revendication 1, comprenant également
une première ouverture (24a) d'une forme globalement rectangulaire et
une seconde ouverture (24b) présentant une forme trapézoïdale.

3. Le collier cervical (10) d'après la revendication 1, comprenant également une ouverture arrière (24c) permettant d'accéder au cou du patient.

4. Le collier cervical (10) d'après la revendication 1, comprenant également un substrat (20), un rembourrage (40) et une fixation (120) ; la fixation (120) possédant deux extrémités, la première d'entre elles étant disposée entre le substrat (20) et le rembourrage (40) et la seconde étant dotée d'un morceau de Velcro.

5. Le collier cervical (10) d'après la revendication 4, le substrat (20) comprenant une partie avant (12a) en forme de V, la première ouverture (24a) étant située au centre de la portion avant (12a) et la seconde ouverture (24b) sur le côté de la partie avant (12a).

6. Le collier cervical (10) d'après la revendication 4, le substrat comprenant une partie arrière (14a), cette partie arrière (14a) comprenant une ouverture arrière (24c), l'ouverture arrière (24c) permettant d'accéder au cou du patient.

7. Le collier cervical (10) d'après la revendication 1, comprenant également un substrat (20) comportant lui-même une partie avant (12a), une partie arrière (14a) et une partie charnière (16a), cette partie charnière (16a) se situant entre la partie avant (12a) et la partie arrière 14a),
la première ouverture (24a) étant située au centre de la partie avant (12a) et la seconde ouverture (24b) sur le côté de la partie avant (12a) ;
une ouverture arrière (24c) permettant d'accéder au cou du patient étant prévue dans la partie arrière (14a).

8. Le collier cervical (10) d'après la revendication 7, la partie arrière (14a) comportant plusieurs encoches (30) sur une bordure (28a, 28b) de la partie arrière (14a).

9. Un système de traitement des patients comprenant un collier cervical (10) d'après la revendication 1 permettant de traiter les patients, le système de traitement comprenant :
un pansement (200) situé dans la seconde ouverture (24b), le pansement (200) comportant une base présentant une surface incurvée (204a) située à proximité du cou du patient au-dessus de la carotide, le pansement (200) contenant un premier agent réactif (220a) et un second agent réactif (220b) ;
le premier agent réactif (220a) et le second agent réactif (220b) créant une réaction thermique lorsqu'ils sont mélangés.

10. Le système de traitement des patients d'après la revendication 9, le pansement (300) comprenant également
un premier conteneur (306) contenant la base (302a) ;
un second conteneur (308) entièrement situé à l'intérieur du premier conteneur (306) ;
un déclencheur (331) à actionner par l' utilisateur ;
un initiateur (332b) qui dans un premier temps sépare le premier agent réactif du second, l'initiateur étant contrôlé par le déclencheur pour permettre le mélange entre le premier agent réactif et le second dans un second temps.

11. Le système de traitement des patients d'après la revendication 10, le premier agent réactif étant sous forme liquide et le second sous forme sèche.

12. Le système de traitement des patients d'après la revendication 11,
le premier agent réactif étant constitué
d'eau 99,890244 % du volume total du premier agent réactif
de propylène glycol 0,104878 % du volume total du premier agent réactif et
de flavonoïde 0,004878 % du volume total du premier agent réactif et
le second agent réactif était constitué
d'hydroxyproline 40,000 % du volume total du second agent réactif,
de sel de sodium d'acide alginique 30,000 % du volume total du second agent réactif et
de bétaïne 30,000 % du volume total du second agent réactif.

13. Le système de traitement des patients d'après la revendication 10, le déclencheur (331) et l'initiateur (332b) étant reliés par une tige (332a).

14. Le système de traitement des patients d'après la revendication 9, comprenant également une fixation (120) pour maintenir le pansement dans la seconde ouverture.

15. Le système de traitement des patients d'après la revendication 14, comprenant également un connecteur (32), un substrat (20) et un rembourrage (40), le connecteur (32) prenant la fixation (120) en sandwich entre le substrat (20) et le rembourrage (40).
